# EUROPEAN PATENT APPLICATION

(11) **EP 2 095 767 A1**
(43) Date of publication of application: **02.09.2009**
(21) Application number: 07851051.8
(22) Date of filing: 21.12.2007
(51) Int. Cl.: A61B 5/157, A61B 5/15, A61B 5/151

(54) **BLOOD SENSOR AND BLOOD INSPECTION DEVICE USING IT**

(30) Priority: 21.12.2006 JP 2006344089; 21.12.2006 JP 2006344090
(71) Applicant: Panasonic Corporation, Kadoma-shi Osaka 571-8501 (JP)
(72) Inventor: AKIYAMA, Toshihiro, Ehime 791-0395 (JP)
(74) Representative: Grünecker, Kinkeldey, Stockmair & Schwanhäusser Anwaltssozietät
(86) International application number: PCT/JP2007/074650
(87) International publication number: WO 2008/075768

(57) **Abstract**

A blood sensor usable both for a blood inspection device that performs puncture with a puncturing needle and for a blood inspection device that performs puncture with laser. In concrete, this invention comprises a sensor unit having a holder and a blood sensor mounted to the holder and a needle unit having a slidable puncturing needle. The needle unit provides a blood sensor block detachably fitted to the inside of the holder of the sensor unit. The blood sensor block is applied to a needle-puncturing type blood inspection device. The sensor unit from which the needle unit is removed is applied to a laser-puncturing type blood inspection device.

## Description

### Technical Field

The present invention relates to a blood sensor and a blood test apparatus to which the blood sensor is attached.

### Background Art

Diabetes patients need to measure their blood sugar level on a regular basis and inject insulin based on the measured blood sugar level to maintain a normal blood sugar level. To maintain this normal blood sugar level, diabetes patients need to measure the blood sugar level on a regular basis, and sample a small amount of blood from their fingertips with a blood test apparatus and measure the blood sugar level from the sampled blood.

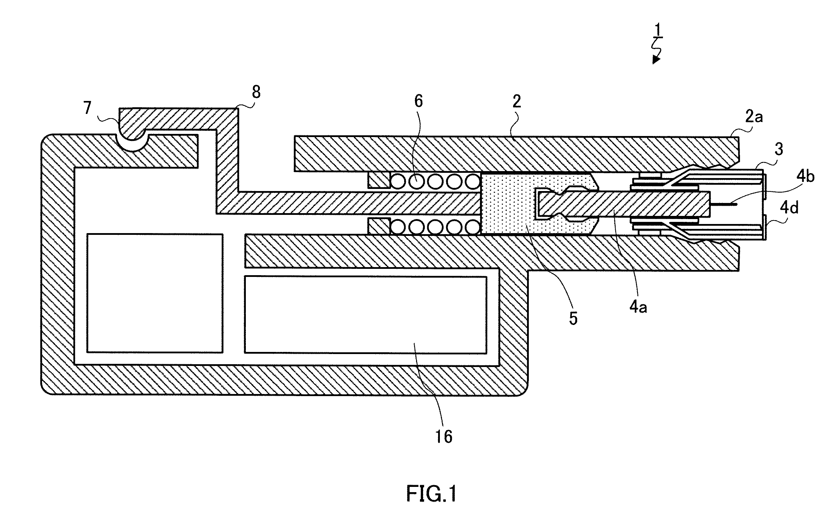

From the standpoint of a puncturing means, blood test apparatuses are broadly classified into apparatuses that perform puncturing by means of a puncturing needle and apparatuses that perform puncturing by means of laser light. FIG.1 shows blood test apparatus 1 that performs puncturing by means of a puncturing needle. Blood test apparatus 1 is composed of: blood sensor block 3 (hereinafter also referred to as "sensor block") that is attached to front end 2a of housing 2; plunger 5 that holds lancet 4a which makes sensor block 3; spring 6 that urges this plunger 5 toward front end 2a; and handle 8 one end of which is attached to plunger 5 and the other end of which engages with locking part 7.

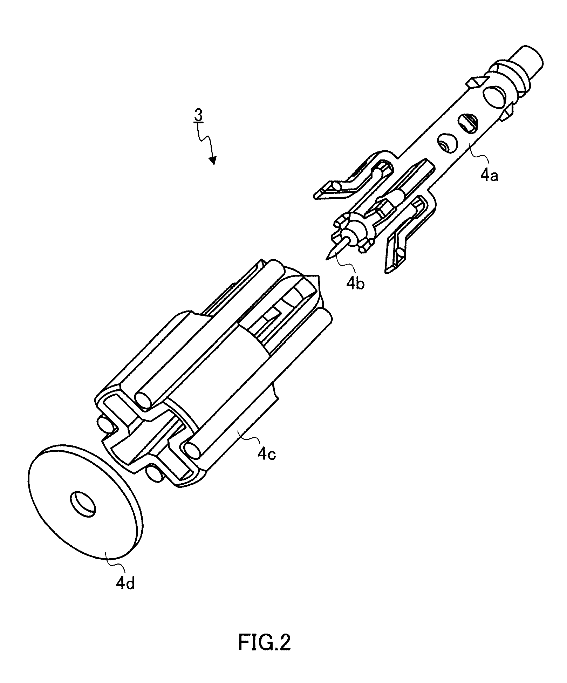

Further, with sensor block 3, as shown in the assembly diagram of FIG.2, lancet 4a to which puncturing needle 4b is attached is provided slidably in holder 4c. Blood sensor 4d (hereinafter "sensor") is attached to the other end of holder 4c. Because puncturing needle 4b needs to be replaced from the viewpoint of health every time puncturing needle 4b is used, puncturing needle 4b and sensor 4d are formed integrally.

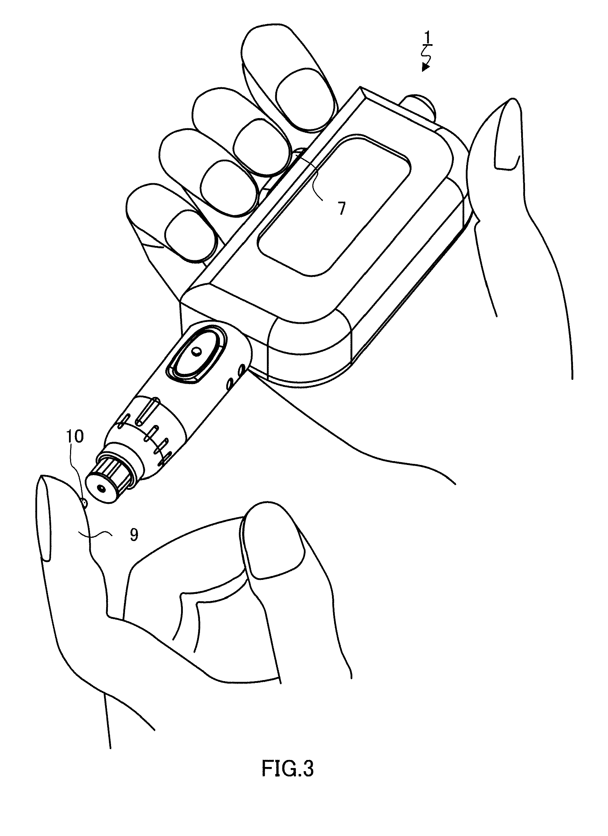

The operation of blood test apparatus 1 composed as described above will be explained below. As shown in FIG.3, for example, blood test apparatus 1 is held by the right hand and is abutted on skin 9 of the left hand. Then, when engagement with locking part 7 shown in FIG.1 is released, plunger 5 urged by spring 6 is launched toward front end 2a. Puncturing needle 4b attached to the other end of lancet 4a passes sensor 4d and punctures skin 9. By this means, blood 10 flows out from skin 9. Blood 10 that has flowed out is detected by blood sensor 4d. Then, electrical circuit section 16 provided in blood test apparatus 1 measures the amount of blood components. After this measurement, sensor block 3 that has been used is removed and discarded.

Blood test apparatus 1 is likely to be advantageous in terms of cost compared to the blood test apparatus (described later) that performs puncturing by means of laser light. However, sensor block 3 used in blood test apparatus 1 has puncturing needle 4b and therefore may also become disadvantageous in terms of cost.

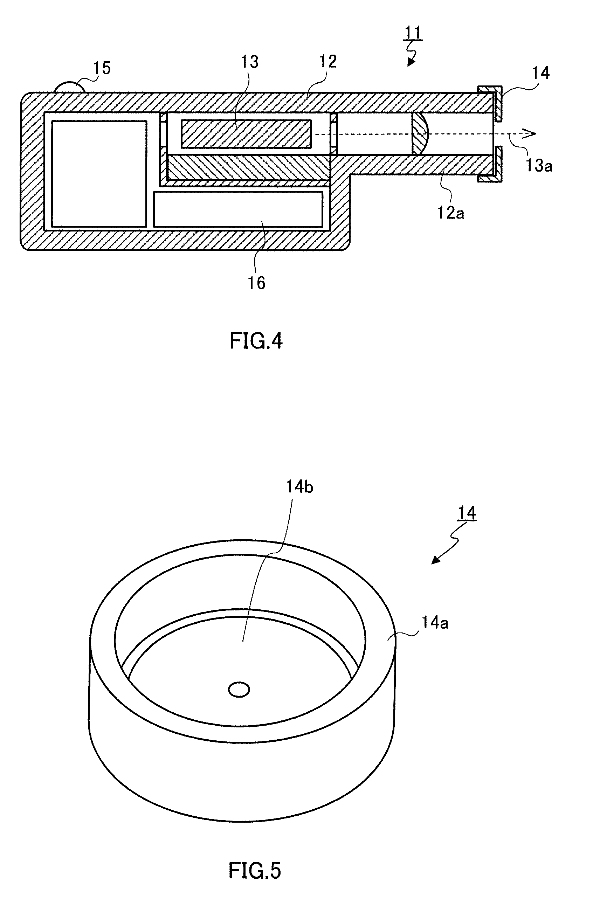

The blood test apparatus that performs puncturing by means of laser light will be explained (see, for example, Patent Document 2). FIG.4 shows blood test apparatus 11 that performs puncturing by means of laser light. For example, blood test apparatus 11 has: housing 12; laser emitting apparatus 13 provided in housing 12; sensor unit 14 attached to front end 12a of housing 12; emission button 15 for emitting laser light 13a from laser emitting apparatus 13; and electrical circuit section 16 connected with laser emitting apparatus 13. As shown in FIG.5, sensor unit 14 is formed integrally by holder 14a and sensor 14b attached in holder 14a. Sensor 14b only needs to be replaced per sensor unit 14, which is convenient.

The operation of blood test apparatus 11 will be explained. When emission button 15 is pressed, laser light 13a is emitted from laser emitting apparatus 13 toward sensor 14b. This laser light 13a passes sensor 14b and punctures skin 9 (see FIG.3). Blood 10 flowing out from punctured skin 9 is detected by sensor 14b and the blood component level is measured in electrical circuit section 16 provided in blood test apparatus 11.

Blood test apparatus 11 has laser emitting apparatus 13, which makes the configuration complex, and is disadvantageous in terms of cost. By contrast with this, sensor unit 14 does not require puncturing needle 4b and is advantageous in terms of cost.
Patent Document 1: Japanese Translation of PCT Application Laid-Open No.2003-524496
Patent Document 2: Japanese Translation of PCT Application Laid-Open No.2004-533866

### Disclosure of Invention

### Problems to be Solved by the Invention

As described above, a sensor block attached to a conventional needle puncturing type blood test apparatus is formed integrally with a blood sensor and a puncturing needle. The blood sensor can be molded with resin and the puncturing needle can be made of metal. Consequently, when a sensor block that has been used is discarded, the blood sensor and the puncturing needle need to be separated. Therefore, the sensor block is removed from the needle puncturing type blood test apparatus, and the lancet, which has the puncturing needle, is discarded separately from the removed sensor block as hazardous components. There are cases where this separation becomes a burden on users (i.e. patients).

Moreover, conventional needle puncturing type blood test apparatuses and laser puncturing type blood test apparatuses both require custom-designed blood sensors. That is, blood sensors that can be used in needle puncturing type or laser puncturing type apparatuses are not compatible with each other. Therefore, in the case where patients who have used needle puncturing type blood test apparatus intend to use laser puncturing type blood test apparatus, they need new kind of blood sensors.

It is therefore an object of the present invention to provide a blood sensor that can be used in both blood test apparatuses that perform puncturing by means of a puncturing needle and blood test apparatuses that perform puncturing by means of laser light. Then, patients can use any blood test apparatus as long as they carry a blood sensor of one kind, and this is more convenient for them.

### Means for Solving the Problem

That is, the first of the present invention relates to a blood sensor unit and a blood sensor block explained below.
[1] A sensor unit has: a holder; and a blood sensor attached to the holder, whereby a needle unit with a puncturing needle slidably provided can be attached detachably inside the holder in the sensor unit.
[2] A blood sensor block has : a sensor unit that includes: a holder; and a blood sensor attached to the holder; and a needle unit with a puncturing needle provided slidably, whereby the needle unit is attached detachably to the holder in the sensor unit.

A second of the present invention relates to a laser puncturing type blood test apparatus to which the sensor unit is attached and a blood test apparatus without a puncturing means.
[3] A blood test apparatus has: a housing that includes at one end an insertion part onto which the sensor unit according to [1] is attached; an electrical circuit section that is provided in the housing and that is connected with a blood sensor in the sensor unit; and puncturing means that is provided in the housing and that emits laser light to puncture skin.
[4] A blood test apparatus has: a housing that includes at one end an insertion part onto which the sensor unit according to [1] is attached; and an electrical circuit section that is provided in the housing and that is connected with the blood sensor in the sensor unit, whereby the blood test apparatus does not have puncturing means in the housing.

A third of the present invention relates to a puncturing needle type blood test apparatus to which the blood sensor block is attached.
[5] A blood test apparatus has: a housing that includes at one end an insertion part in which the blood sensor block according to [2] is attached; an electrical circuit section that is provided in the housing and that is connected with the blood sensor in the sensor unit; and puncturing means that is provided in the housing and that drives the puncturing needle of the needle unit to puncture skin.

### Advantageous Effect of the Invention

While a puncturing needle unit is attached detachably to the blood sensor unit according to the present invention, the blood sensor unit can also be attached to laser puncturing type blood test apparatuses and blood test apparatuses without a puncturing means. Further, the blood sensor block in which the puncturing needle unit is attached to the blood sensor unit can be attached to needle puncturing type blood test apparatuses. In this way, the blood sensor unit according to the present invention can be used in both blood test apparatuses that perform puncturing by means of a puncturing needle and blood test apparatuses that perform puncturing by means of laser light. Accordingly, even when patients who use blood test apparatuses that perform puncturing by means of a puncturing needle use blood test apparatuses that perform puncturing by means of laser light, it is possible to use the blood sensor in laser puncturing type blood test apparatuses without wasting the blood sensor. That is, unused blood sensors are not wasted, which is economical.

Further, the sensor unit and the puncturing needle unit of the blood sensor block according to the present invention are individually attached to and removed from needle puncturing type blood test apparatuses, so that it is not necessary to separate the sensor unit and puncturing needle unit after they are removed.

### Brief Description of Drawings

FIG.1 is a cross-sectional view of a conventional needle puncturing type blood test apparatus;
FIG.2 is a perspective view of an assembly of a blood sensor block of a blood test apparatus shown in FIG.1;
FIG.3 illustrates the state of use of the blood test apparatus shown in FIG.1;
FIG.4 4 is a cross-sectional view of a conventional laser puncturing type blood test apparatus;
FIG.5 is a perspective view of a sensor unit of the blood test apparatus shown in FIG.4;
FIG.6 is a conceptual diagram of blood sensor blocks and blood test apparatuses according to the present invention;
FIG.7 is cross-sectional views of a needle unit (FIG.7A), a sensor unit (FIG.7B) and blood sensor block (FIG.7C);
FIG.8 is a top view of the blood sensor block according to the present invention;
FIG.9 is a partial development view of members that guide the needle unit such that the needle unit is inserted in the sensor unit of the blood sensor block according to the present invention;
FIG. 10 is a partial development view of members that guide the sensor unit such that the sensor unit is inserted in the blood test apparatus according to the present invention;
FIG.11 is a plan view of the blood sensor block according to the present invention seen from the blood sensor side;
FIG. 12 is a cross-sectional view of the blood sensor;
FIG. 13 is a perspective plan view of the blood sensor;
FIG.14 is a plan view (FIG.14A) of a cover that constitutes the blood sensor, a plan view (FIG.14B) of a spacer and a plan view of a substrate (FIG.14C);
FIG. 15 is a cross-sectional view near a storing part of the blood sensor;
FIG.16 is a plan view near the storing part of the blood sensor;
FIG.17 shows the state where blood flowing out from punctured skin is taken in by the blood sensor;
FIG.18 is a cross-sectional view of the first needle puncturing type blood test apparatus;
FIG.19 is a block diagram of an electrical circuit of the first needle puncturing type blood test apparatus;
FIG.20 illustrates the flowchart of blood test in the first needle puncturing type blood test apparatus;
FIG. 21 is a cross-sectional view of the second needle puncturing type blood test apparatus;
FIG.22 is a cross-sectional view near an insertion part of the second needle puncturing type blood test apparatus;
FIG.23 is a plan view of the blood sensor block of the second needle puncturing type blood test apparatus;
FIG.24 is a cross-sectional view showing the state where the sensor unit is removed from the insertion part of the second needle puncturing type blood test apparatus;
FIG.25 is a cross-sectional view showing the state where the needle unit is removed from the insertion part of the second needle puncturing type blood test apparatus;
FIG.26 is a cross-sectional view of the laser puncturing type blood test apparatus;
FIG.27 is a block diagram of the electrical circuit of the laser puncturing type blood test apparatus;
FIG.28 illustrates the flowchart of blood test with the laser puncturing type blood test apparatus; and
FIG.29 is a cross-sectional view of the blood test apparatus without a puncturing means.

### Best Mode for Carrying Out the Invention

Hereinafter, an embodiment of the present invention will be explained based on the drawings.

### 1. Blood sensor unit and blood sensor block

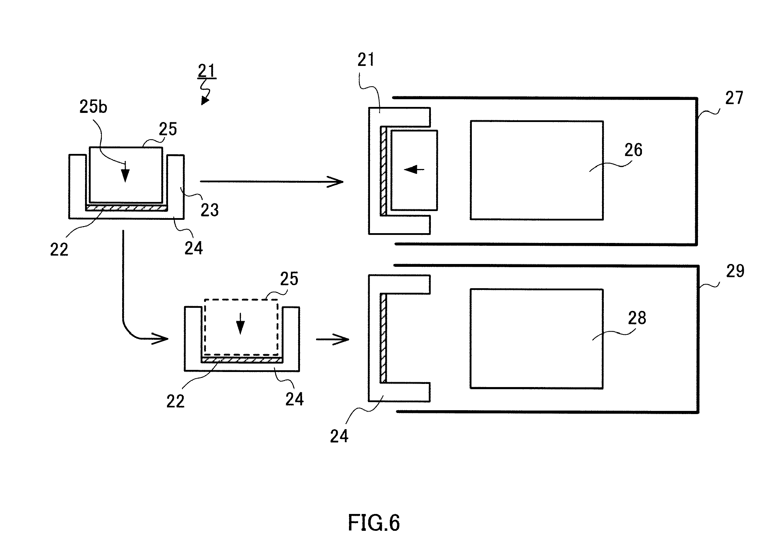

FIG.6 is a conceptual diagram of blood sensor block 21 (hereinafter also referred to as "sensor block") according to the present invention and a blood test apparatus to which sensor block 21 is attached. Sensor block 21 shown in FIG.6 is composed of sensor unit 24 and needle unit 25 that is detachably attached to sensor unit 24. Sensor unit 24 is composed of sensor 22 and holder 23 to which sensor 22 is attached.

Sensor block 21 is detachably attached to blood test apparatus 27 (hereinafter also referred to as "needle puncturing type blood test apparatus") that uses puncturing means 26 for driving puncturing needle 25b of needle unit 25. On the other hand, sensor unit 24 to which needle unit 25 is not attached is detachably attached to blood test apparatus 29 (hereinafter also referred to as "laser puncturing type blood test apparatus") that performs puncturing by means of laser light with laser emitting apparatus 28.

Needle unit 25 is detachably attached to sensor unit 24, and, consequently, can be applied to any one of needle puncturing type blood test apparatus 27 or laser puncturing type blood test apparatus 29 depending on whether or not needle unit 25 is attached to sensor unit 24.

Consequently, even when users (for example, patients) of a blood test apparatus change their apparatus from needle puncturing type blood test apparatus 27 to laser puncturing type blood test apparatus 29, they can apply the same sensor block 21 to laser puncturing type blood test apparatus 29. That is, when there is a stock of sensor blocks 21, sensor units 24 of sensor blocks 21 can be used without wasting sensor blocks 21 entirely.

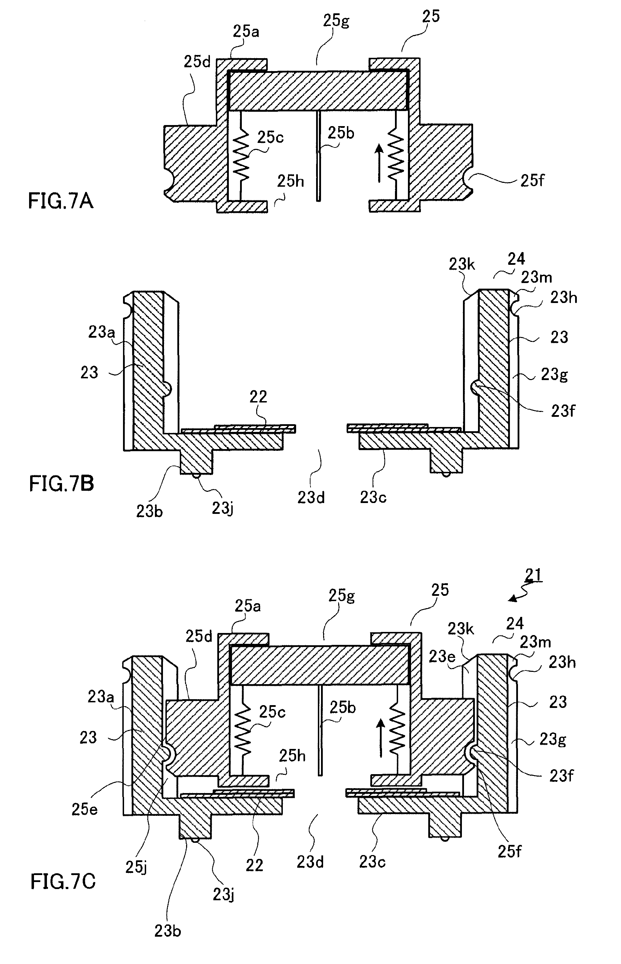

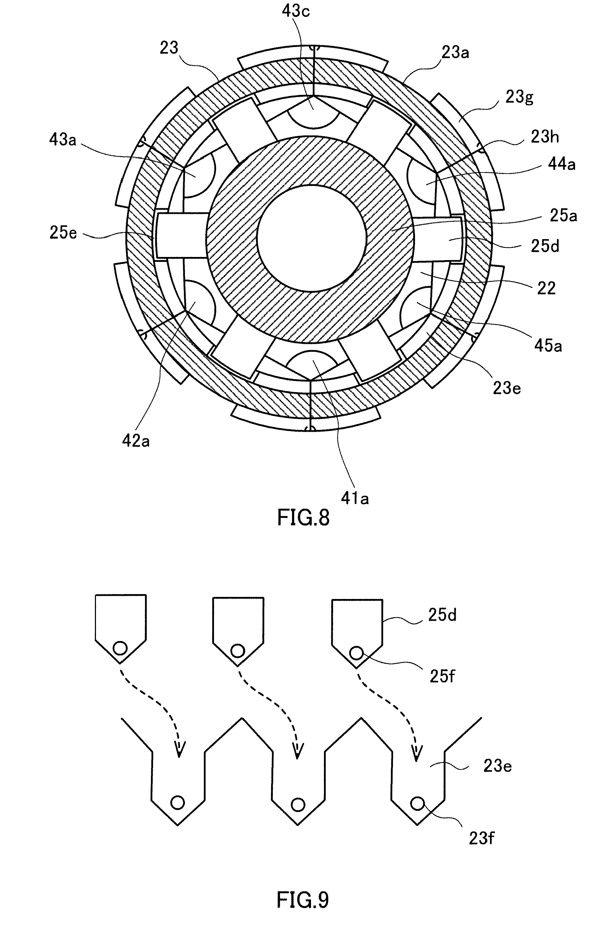

FIG.7A is a cross-sectional view of needle unit 25, FIG.7B is a cross-sectional view of sensor unit 24 and FIG.7C is a cross-sectional view of sensor block 21. FIG.8 is a top view of sensor block 21 seen from above in FIG.7C. As shown in FIG.7C, sensor block 21 has sensor unit 24 and needle unit 25 that is detachably attached inside sensor unit 24 from above in FIG.7C.

Sensor unit 24 is composed of holder 23 and blood sensor 22 that is attached to holder 23. Preferably, holder 23 and blood sensor 22 are formed integrally, that is, formed such that they cannot be separated (unless destroyed). Holder 23 is a member that is open on both ends and is made of synthetic resin and the like. Holder 23 may be shaped cylindrically or may be a cylindrical body having an oval or polygonal cross-section.

Holder 23 is integrally formed with: cylindrical upper part 23a; cylindrical lower part 23b having a smaller diameter than cylindrical upper part 23a; and circular disc-shaped receiving part 23c that partitions between cylindrical lower part 23b and cylindrical upper part 23a. Hole 23d is formed in the center of receiving part 23c.

The surface of holder 23 is preferably water-repellant. The entire surface of holder 23 may be water-repellant, and preferably for example, the surface that contacts skin is preferably water-repellant and, by this means, blood flowed out from skin is prevented from leaking out to outside holder 23.

Preferably, the dimensions of holder 23 are set such that lower part 23b of holder 23 in the second sensor unit can be inserted inside upper part 23a of holder 23 in the first sensor unit. A plurality of sensor units can be stacked in layers and accommodated, and can be accommodated in small space because holder 23 in the first sensor unit and holder 23 in the second sensor unit are overlaid.

Preferably, at least part of holder 23 is transparent to allow the interior to be seen from outside. For example, preferably, holder 23 is made transparent to allow the blood storing part of blood sensor 22 to be seen in order to visually check the state of blood sampling.

Preferably, the first insertion guides for guiding insertion of needle unit 25 are arranged on the inner wall surface of cylindrical upper part 23a of holder 23 of sensor unit 24. The first insertion guides are, for example, guide grooves 23e (see FIG.7C and FIG.8). Positioning convex parts 23f are formed in lower portions of guide grooves 23e inside holder 23 composing sensor unit 24 shown in FIG.7B and FIG.7C. Needle unit 25 guided by guide grooves 23e and inserted into sensor unit 24 is positioned by convex parts 23f and attached.

By contrast with this, second insertion guides for guiding insertion to the blood test apparatus (either needle puncturing type 27 or laser puncturing type 29) are preferably arranged on the outer wall surface of cylindrical upper part 23a of holder 23 in sensor unit 24. The second insertion guides are, for example, guides 23g of a convex shape. Positioning concave parts 23h are formed in upper portions of guides 23g on the outer surface of holder 23 composing sensor unit 24 shown in FIG.7B and FIG.7C. The sensor unit guided by guides 23g and inserted in the blood test apparatus is positioned by concave parts 23h and attached.

Needle unit 25 has: holder 25a; puncturing needle 25b of a thumbtack shape provided slidably inside holder 25a and elastic bodies 25c (for example, leaf springs) that urge puncturing needle 25b upward in FIG.7. Holder 25a is a cylindrical member made of resin. The elastic bodies that urge puncturing needle 25b upward may be coil springs or springs.

A plurality of guide plates 25d are arranged at equal intervals in the outer wall surface of holder 25a in needle unit 25. Guide plates 25d mesh with guide grooves 23e (i.e. first insertion guides) provided in the inner wall surface of holder 23 in sensor unit 24. By this means, needle unit 25 is guided and inserted inside holder 23 in sensor unit 24 and is attached detachably and slidably in holder 23.

Positioning concave parts 25f are provided in lower portions of guide plates 25d of needle unit 25. Positioning concave parts 25f engage with positioning convex parts 23f provided in the inner wall surface of holder 23 in sensor unit 24 and determine the position to attach needle unit 24.

Further, the number of guide plates 25d provided in holder 25a is determined based on the shape of blood sensor 22 of sensor unit 24 and the number of connection electrodes arranged in blood sensor 22. As shown in FIG. 8, when blood sensor 22 is hexagonal and connection electrodes 41a to 45a and 43c are formed in the apexes of the regular hexagon, six guide plates 25d is required to be provided (see FIG.8).

When needle unit 25 is inserted in sensor unit 24, guide plates 25d provided in needle unit 25 are guided as shown in FIG.9 along guide grooves 23e provided in sensor unit 24. Accompanying this, the rotation angle of needle unit 25 is adjusted so that needle unit 25 is guided to a predetermined position. When needle unit 25 is guided to the predetermined position, positioning convex parts 23f of sensor unit 24 engage with positioning concave parts 25f of needle unit 25 and are positioned, thereby attaching needle unit 25 to sensor unit 24.

When needle unit 25 is attached in the predetermined position in sensor unit 24, connection electrodes 41a to 45a and 43c of blood sensor 22 in sensor unit 24 are exposed without being covered by guide plates 25d of needle unit 25. Connectors (described later) of the blood test apparatus approach to contact exposed connection electrodes 41a to 45a and 43c from above in FIG.8. By this means, connection electrodes 41a to 45a and 43c of blood sensor 22 and electrical circuit section 52 (described later) of the blood test apparatus are connected, so that blood test is possible.

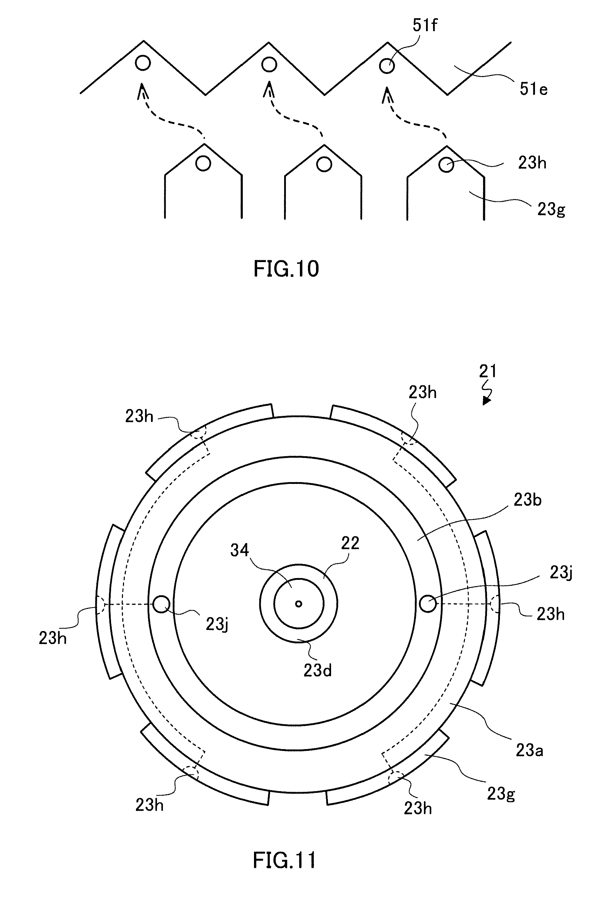

Further, as shown in FIG.10, when sensor unit 24 is inserted in a blood test apparatus (either needle puncturing type 27 or laser puncturing type 29), guides 23g are guided to predetermined positions along guide grooves 51e (described later) formed in the blood test apparatus. Positioning concave parts 23h of guides 23g guided to the predetermined positions engage with positioning convex parts 51f of guide grooves 51e and are positioned. In this way, sensor unit 24 is attached to the blood test apparatus while adjusting the angle of sensor unit 24. Consequently, insertion of sensor unit 24 (or sensor block 21 including sensor unit 24) to the blood test apparatus becomes easy. For example, even when sensor unit 24 (or sensor block 21) is inserted in the blood test apparatus at an arbitrary angle, the connectors of the blood test apparatus can contact connection electrodes 41a to 45a and 43c in a reliable manner.

Further, as shown in FIG.7 and FIG.8, circular hole 25g is formed above holder 25a in needle unit 25 in FIG.7 and circular hole 25h is formed below holder 25a in FIG.7. When puncturing needle 25b is hit by puncturing means 26 (described later) which has passed through hole 25g in blood test apparatus 27, puncturing needle 25b moves downward in FIG.7. Puncturing needle 25b that has moved passes hole 25h and storing part 34 (described later) of blood sensor 22 to puncture skin 9.

Preferably, the diameter of holder 25a of needle unit 25 is greater than the diameter of hole 23d provided in receiving part 23c in sensor unit 24. Receiving part 23c receives puncturing needle 25b that has been hit by puncturing means 26 of blood test apparatus 27 and has moved downward, so that load upon sensor 22 is reduced, thereby preventing deformation of sensor 22.

Preferably, as shown in FIG.7C, positioning concave parts 23h of sensor unit 24 and positioning concave parts 25f of needle unit 25 are spaced apart as much as possible. Positioning convex parts 23f in sensor unit 24 engage with positioning concave parts 25f of needle unit 25 and positioning concave parts 23h engage with positioning convex parts (described later) in the cylinder body of the apparatus. Therefore, when sensor unit 24 is removed from the apparatus, engagement between convex parts and concave parts in the cylinder body of the apparatus is released, and also the engagement between convex parts 23f and concave parts 25f is released. Releasing these engagements produces stress with which sensor unit 24 and needle unit 25 get distorted. Consequently, concave parts 23h and concave parts 25f are spaced apart as much as possible to spread the stress.

In the case where concave parts 23h are provided in upper portions in sensor unit 24, the cover part (described later) of the apparatus can cover sensor unit 24, so that it is possible to hold sensor unit 24 more stably. On the other hand, in the case where concave parts 25f are provided in lower portions in needle unit 25, the engaging parts of needle unit 25 and sensor unit 24 are positioned deeper inside holder 23 in sensor unit 25, so that needle unit 25 is held more stably.

Inclining parts 25j are provided in lower portions of guide plates 25d and inclining parts 23k are provided in upper portions in the inner surface of upper part 23a of holder 23. Inclining parts 25j and 23k make it easier to insert needle unit 25 into sensor unit 24. Similarly, it may also be possible to make it easier to insert sensor unit 24 into the blood test apparatus by providing inclining parts 23m in upper portions in the outer surface of upper part 23a of holder 23 and inclining parts (not shown) in blood test apparatus 27.

Skin detecting sensors 23j may be provided in lower part 23b of holder 23 in sensor unit 24. Preferably, skin detecting sensors 23j make a pair of electrical conductive electrodes in terms of lower cost but may be optical sensors or temperature sensors.

FIG.11 is a plan view of sensor block 21 in FIG.7C seen from the bottom in FIG.7C. As shown in FIG.11, in the lower end of lower part 23b of holder 23, skin detecting sensors 23j of two electrical conductive electrodes (i.e. a pair of electrodes) are provided 180 degrees apart. Contact with skin is detected through changes of the resistance value between a pair of the electrical conductive electrodes upon contacting the electrodes with the skin. A plurality of skin detecting sensors 23j (a plurality of pairs of electrodes) may be provided symmetrically. Detectability of contacting skin improves through increasing the number of skin detecting sensors 23j.

Preferably, as shown in FIG.11, the conductive electrodes of skin detecting sensors 23j are connected through copper interconnections (see the dotted lines) to positioning concave parts 23h formed in guides 23g. One conductive electrode is connected with three adjacent positioning concave parts 23h and the other conductive electrode is connected with other three adjacent positioning concave parts 23h. By this means, the blood test apparatus detects signals (i.e. changes in the resistance value in this case) from two conductive electrodes and detects contact of skin.

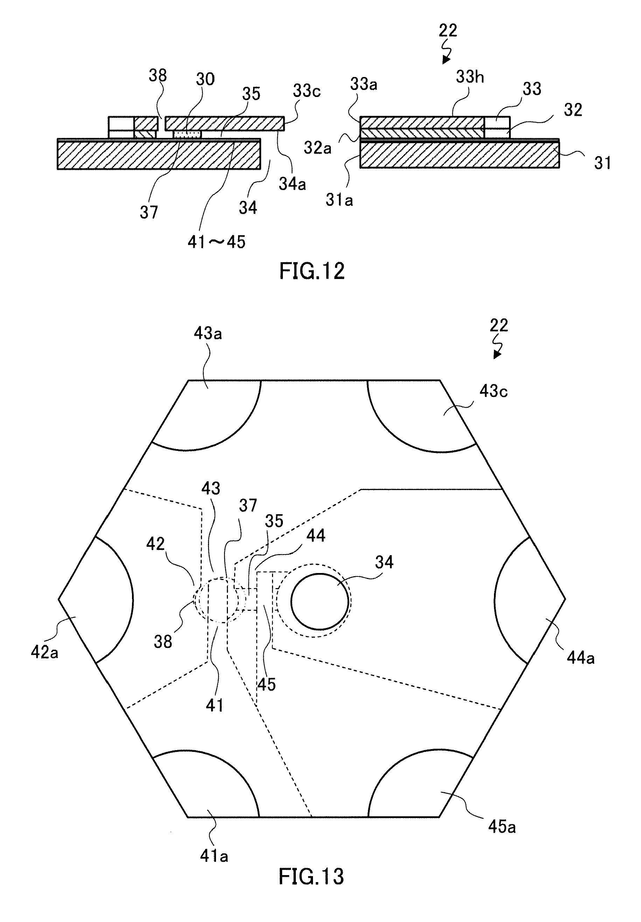

FIG.12 is a cross-sectional view of blood sensor 22 attached to sensor unit 24. Blood sensor 22 has a plate shape and is composed of: substrate 31; spacer 32 laminated on the upper surface of substrate 31; and cover 33 laminated on the upper surface of spacer 32.

Substrate hole 31a formed in practically the center of substrate 31, spacer hole 32a formed in practically the center of spacer 32 and cover hole 33a formed in practically the center of cover 33 communicate, to constitute blood storing part 34.

Blood from punctured skin is sampled in storing part 34 by abutting the lower surface of substrate 31 on skin. Therefore, storing part 34 is open downward. Storing part 34 and air hole 38 are communicated through supply channel 35. The blood stored in storing part 34 flows in supply channel 35 by capillary action and is led to detecting section 37 arranged in supply channel 35.

Upper surface 33h of cover 33 is preferably water-repellant, and, on the other hand, the inner surface of supply channel 35 is preferably hydrophillic. Ceiling 34a of storing part 34 is preferably less hydrophilic than supply channel 35 or less water-repellent than upper surface 33h of cover 33.

Reagent 30 is arranged on detecting section 37. Reagent 30 can be arranged by dropping and drying the reagent 30 solution on detection electrode 41 and detection electrode 43 (see FIG.13) formed in substrate 31.

FIG.13 is a perspective plan view of blood sensor 22. Blood sensor 22 is a regular hexagon (a circular or polygonal shape is also possible). Connection electrodes 41a to 45a, and reference electrode 43c that conducts with connection electrode 43a, are arranged in respective apexes of the regular hexagon. When sensor unit 24 is attached to the blood test apparatus (either needle puncturing type 27 or laser puncturing type 29), connection electrodes 41a to 45a and connection electrode 43a are connected with the connectors (described later) of the blood test apparatus.

Supply channel 35 is provided such that one end of supply channel 35 is connected with storing part 34 provided in practically the center of blood sensor 22. Reagent 30 (see FIG.12) is arranged on detection electrode 41 and detection electrode 43.

Further, the other end of supply channel 35 is communicated to air hole 38. On supply channel 35, there are, from the side closer to storing part 34, detection electrode 44 connected with connection electrode 44a; detection electrode 45 connected with connection electrode 45a; detection electrode 44, which is provided again, connected with connection electrode 44a; detection electrode 43 connected with connection electrode 43a and reference electrode 43c; detection electrode 41 connected with connection electrode 41a; detection electrode 43, which is provided again, connected with connection electrode 43a and reference electrode 43c; and detection electrode 42 connected with connection electrode 42a.

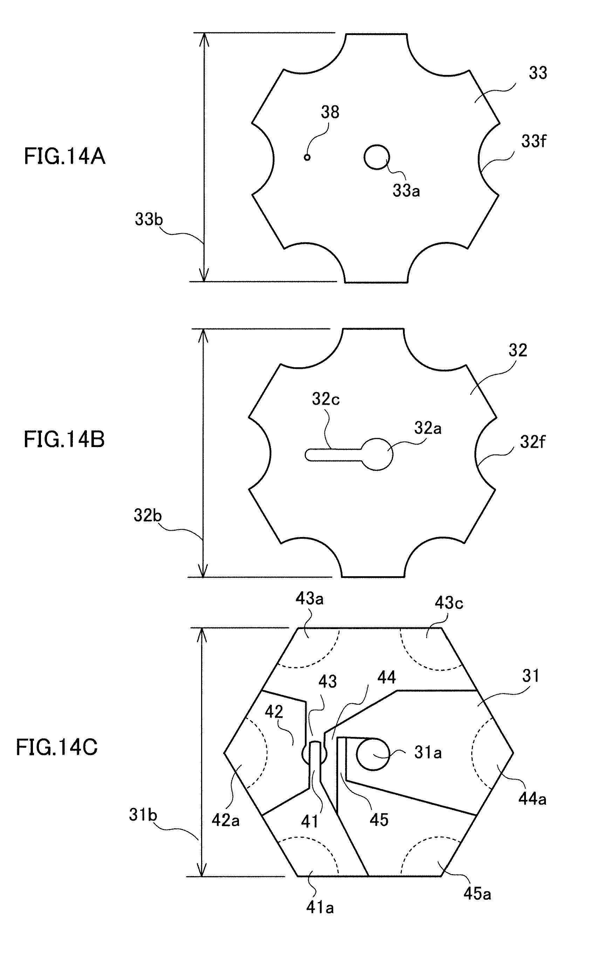

FIG.14A to C are exploded plan views of blood sensor 22. Although blood sensor 22 shown in FIG.14A to C is a regular hexagon, a circular shape or other regular polygonal shape may be possible.

FIG.14C is a plan view of substrate 31 composing blood sensor 22. Substrate hole 31a is provided in practically the center of substrate 31. Substrate 31 is a regular hexagon and its dimension 31b is about 9 mm. The material of substrate 31 can be polyethylene terephthalate (PET) and the thickness of substrate 31 can be about 0.1 mm.

On the upper surface of substrate 31, detection electrodes 41 to 45 and connection electrodes 41a to 45a and reference electrode 43c derived from detection electrodes 41 to 45 are integrally formed. These electrodes may be formed by applying laser machining to the conductive layer (the layer made of gold, platinum, or palladium) formed on the upper surface of substrate 31. The conductive layer may be formed using the sputtering method or the vapor deposition method.

FIG.14B is a plan view of spacer 32 and its dimension 32b is about 9 mm. The material of spacer 32 is polyethylene terephthalate and its thickness is about 0.05 mm. Spacer hole 32a is provided in practically the center of spacer 32. Spacer hole 32a is provided in a position corresponding to substrate hole 31a. This spacer 32 is a regular hexagon and fan-shaped notches 32f are formed in the six apexes of the regular hexagon. Notches 32f are formed in positions corresponding to connection electrodes 41a to 45a and reference electrode 43c of substrate 31.

Slit 32c continuing from spacer hole 32a is formed. Slit 32c becomes blood supply channel 35. Consequently, the wall surfaces of slit 32c and the upper surface of substrate 31 corresponding to the wall surfaces of slit 32c are preferably subjected to hydrophilic treatment.

The width of slit 32c is about 0.6 mm, the length of slit 32c is about 2.5 mm and the cavity of supply channel 35 is about 0.5 microliters. The amount of sampling of blood that is required for test can be reduced, so that it is possible to alleviate burden and fear of patients.

FIG.14A is a plan view of cover 33. Dimension 33b is about 9 mm. The material of cover 33 is polyethylene terephthalate and the thickness of cover 33 is about 0.1 mm. Cover hole 33a is provided in a position slightly decentered from the center of cover 33. Cover 33 is a regular hexagon, and six semicircular notches 33f are formed in positions each corresponding to connection electrodes 41a to 45a and reference electrode 43c of substrate 31 in the apexes of the regular hexagon.

The position of air hole 38 meets the front end part of slit 32c. The diameter of air hole 38 is about 50 micrometers. The diameter of air hole 38 is reduced to prevent blood from flowing out from air hole 38.

Substrate 31, spacer 32 and cover 33 making blood sensor 22 can be formed by dividing a parent substrate of the regulation siz into several pieces. If substrate 31, spacer 32 and cover 33 are made regular hexagons, the parent substrate can be divided efficiently without waste to obtain substrate 31, spacer 32 and cover 33.

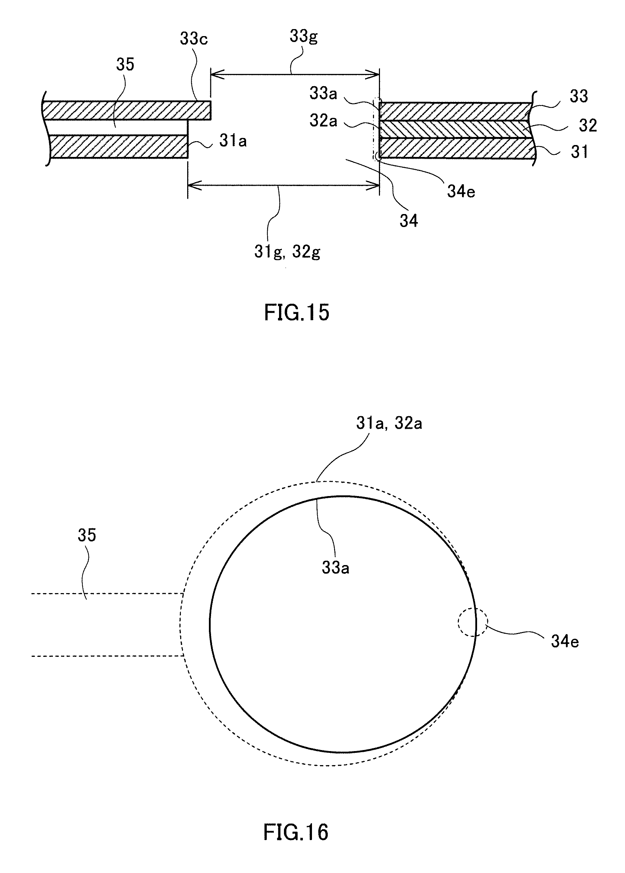

FIG.15 is a cross-sectional view near storing part 34 of blood sensor 22 shown in FIG.12 and FIG.16 is a plan view of storing part 34. As shown in FIG. 15 and FIG. 16, diameter 31g of substrate hole 31a and diameter 32g of spacer hole 32a are the same (about 2 mm), and the center of substrate hole 31a and the center of spacer hole 32a are in the same position.

By contrast with this, diameter 33g of cover hole 33a is about 1.75 mm and is smaller than diameter 31g of substrate hole 31a and diameter 32g (about 2 mm) of spacer hole 32a. The center of cover hole 33a is slightly farther away from supply channel 35 than the centers of substrate hole 31a and spacer hole 32a.

In this way, cover 33 has projecting part 33c projecting from supply channel 35 toward the center of storing part 34. The length of projection of projecting part 33c is about 0.25 mm and is greater 0.1 mm than the sum (0.15 mm) of the thickness of substrate 31 and the thickness of spacer 32.

By contrast with this, on opposite side 34e of supply channel 35 in storing part 34, cover hole 33a, spacer hole 32a and substrate hole 31a are aligned. That is, there are the centers of substrate hole 31a and spacer hole 32a in the center of storing part 34 and the center of cover hole 33a is in a position farther away from supply channel 35 than the center of storing part 34.

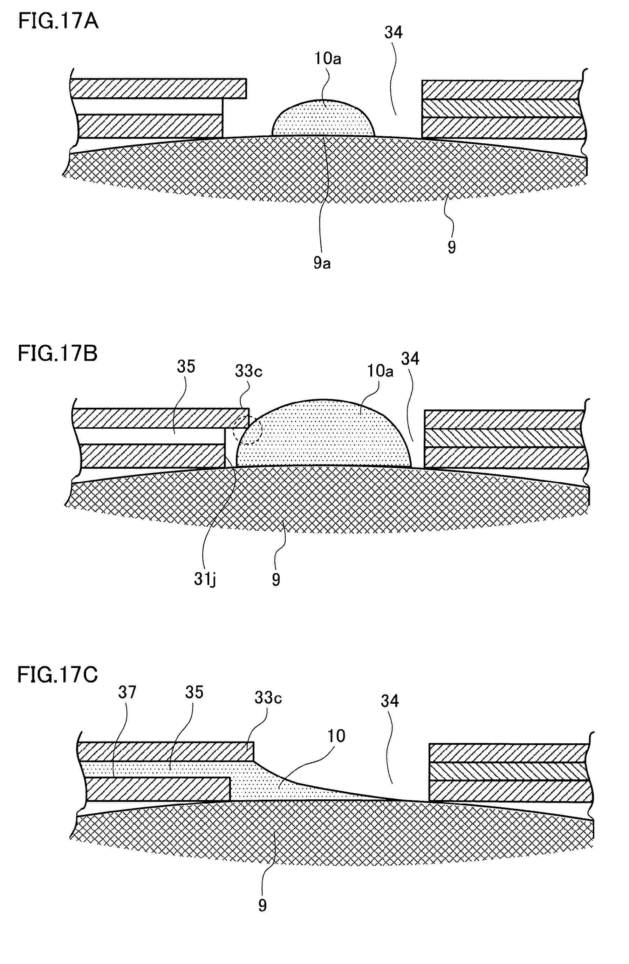

The state where blood 10 is sampled in storing part 34 of blood sensor 22 is shown in FIG.17A to C. As shown in FIG.17A, when skin 9 in the area of storing part 34 is punctured, blood flows out from punctured hole 9a to form blood drop 10a. As shown in FIG.17B, accompanying the outflow of blood, blood drop 10a becomes bigger, and contacts the tip of projecting part 33c (shown by the dotted line). Preferably, blood flows into supply channel 35 before blood drop 10a becomes bigger to contact point 31j of substrate 31 on the supply channel 35 side (see FIG.17C). Blood flows into supply channel 35 at a burst in a rate-controlled state by the capillary action generated by projecting part 33c and skin 9. Blood 10 that has flowed into supply channel 35 is led to detecting section 35.

Capillary action is generated in the space between cover 33 and skin 9 near supply channel 35 of storing part 34. Consequently, blood 10 sampled in storing part 34 flows into supply channel 35 in a reliable manner before blood 10 fills storing part 34, and is led to detecting section 37. Consequently, it is possible to reduce the amount of blood left in storing part 34 and reduce the amount of sampling blood 10. By this means, the burden upon patients is alleviated.

### <Needle puncturing type blood test apparatus 1>

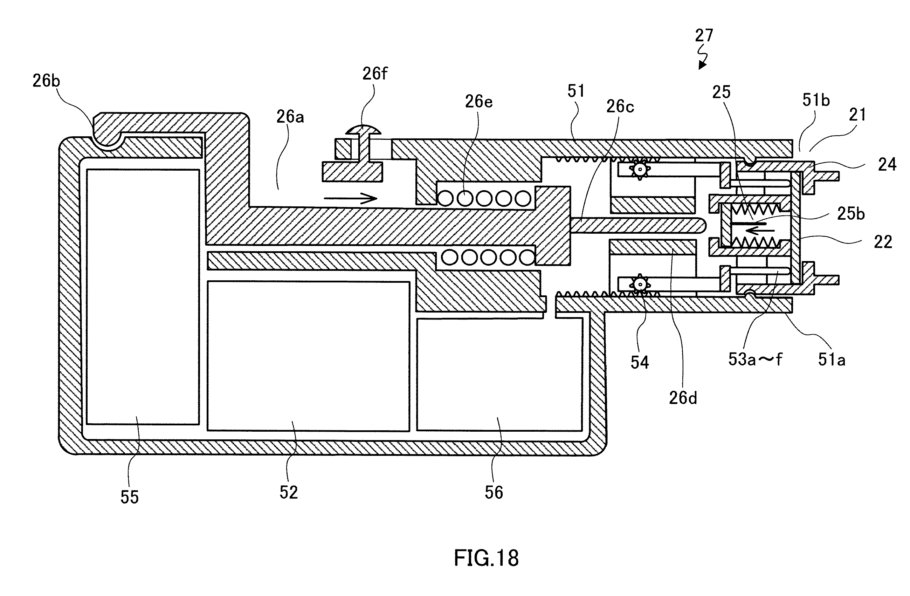

Needle puncturing type blood test apparatus 27 to which sensor block 21 according to the present invention is attached will be explained. FIG.18 is a cross-sectional view of blood test apparatus 27. One end of housing 51 made of resin is cylindrical body 51a of a cylindrical shape. Sensor block 21 is inserted into insertion part 51b formed at the front end of cylindrical body 51a.

As described above, sensor block 21 is composed of: sensor unit 24 of a cylindrical shape to which blood sensor 22 is attached; and needle unit 25 which is inserted in sensor unit 24.

Puncturing needle 25b attached in needle unit 25 faces blood sensor 22. Further, guides 23g formed in the outer surface of holder 23 composing sensor unit 24 (see FIG.7 and FIG.8) are guided along guide grooves 51e formed in the inner surface of cylindrical body 51a, so that sensor block 21 is inserted in cylindrical body 51a. Positioning concave parts 23h formed in guides 23g of sensor block 21 inserted in cylindrical body 51a engage with positioning convex parts 51f formed deep inside guide grooves 51e, so that sensor block 21 is positioned and attached (see FIG.10).

Needle puncturing means 26 (formed with 26a to 26f) of blood test apparatus 27 will be explained. Handle 26a is provided slidably in housing 51. Handle 26a is urged toward sensor block 21 by elastic bodies (for example, springs 26e). One end of handle 26a is led outside housing 51 and is locked by locking part 26b. Further, the other end of handle 26a is coupled to stick 26c facing puncturing needle 25b. Stick 26c can slide in guide 26d arranged in cylindrical body 51a.

When the lock by locking part 26b is released, stick 26c of puncturing means 26 hits puncturing needle 25d by the restoring force of spring 26e. Puncturing needle 25b hit by stick 26c passes storing part 34 of sensor 22 (see FIG.12) and punctures skin.

Puncturing depth adjusting knob 26f moves left and right in FIG.18 to adjust to what extent handle 26a returns, to adjust the puncturing depth of puncturing needle 25b.

Electrical circuit section 52 provided in housing 51 is connected with connectors 53 (53a to 53f). Power is supplied from battery 55 to electrical circuit section 52.

Connectors 53 (including 53a to 53f) contact connection electrodes 41a to 45a and 43c formed in blood sensor 22 (see FIG.13) to connect blood sensor 22 and electrical circuit section 52. When gears 54 rotate, connectors 53 move in the left or right directions in FIG.18. Electrical circuit section 52 can control the rotation of gears 54. By moving connectors 53, it is possible to connect connectors 53 with the electrodes of blood sensor 22 in a reliable manner.

Vacuuming means 56 vacuums the interiors of storing part 34 and lower part 23b of holder 23 composing sensor unit 24 through storing part 34 and air hole 38 of sensor 22.

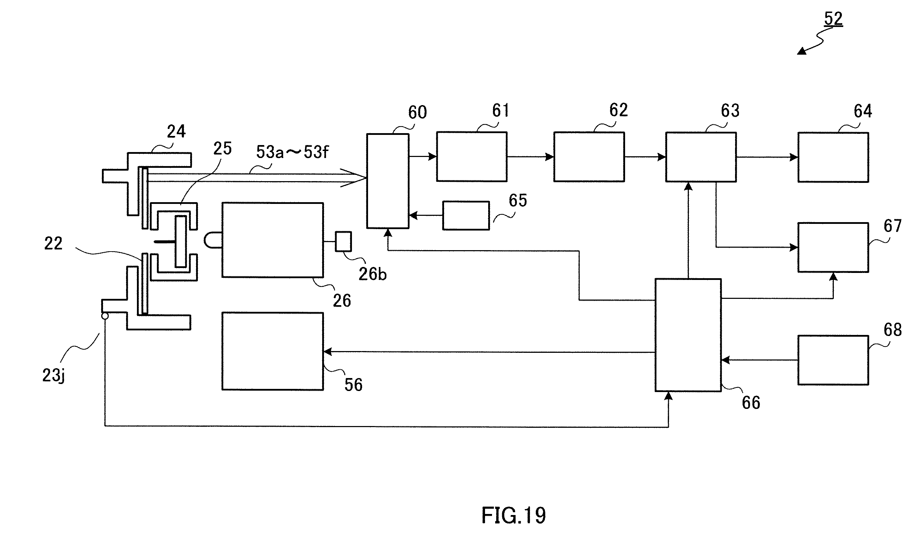

FIG.19 is a block diagram of electrical circuit section 52 of needle puncturing type blood test apparatus 27. Connection electrodes 41a to 45a and reference electrode 43c (see FIG. 13) of blood sensor 22 are connected with switching circuit 60 through connectors 53a to 53f. The output of switching circuit 60 is connected with the input of current/voltage converter 61. The output of current/voltage converter 61 is connected with the input of calculating section 63 through analogue/digital converter 62 (hereinafter "A/D converter"). The output of calculating section 63 is connected with display section 64 (for example, a liquid crystal display) and communication section 67. Further, reference voltage source 65 is connected with switching circuit 60. Reference voltage source 65 may be a ground potential.

The output of controlling section 66 is connected with the controlling terminal of switching circuit 60, calculating section 63, communication section 67, vacuuming means 56 and the driving section (not shown) of gears 54. On the other hand, the input of controlling section 66 is connected with skin detecting sensors 23j and timer 68. Puncturing means 26 is provided facing needle unit 25. Instead of arranging skin detecting sensors 23j, a manual button for starting vacuuming may be arranged.

The operation of electrical circuit section 52 will be explained. Before measurement, connectors 53a to 53f abut on connection electrodes 41a to 45a and reference electrode 43c of blood sensor 22 of attached sensor block 21. By driving gears 54, connectors 53a to 53f move to abut on each electrode.

To which connectors 53a to 53f connection electrodes 41a to 45a and reference electrode 43c of blood sensor 22 are connected is specified. According to a command from controlling section 66, a connector having zero electrical resistance with respect to the adjacent connectors is detected among connectors 53a to 53f. The electrode connected with the connector having zero electrical resistance is determined as connector 53 connected with reference electrode 43c. It is determined based on connector 53 connected with reference electrode 43c that connectors 53 (i.e. in order, starting with any of connectors 53a to 53f) are connected with connection electrodes 44a, 45a, 41a, 42a and 43a, respectively.

The amount of blood components (for example, glucose) is measured by firstly switching switching circuit 60 first to connect detection electrode 41, which serves as an active electrode for measuring the amount of blood components, with current/voltage converter 61. Further, detection electrode 42, which serves as a sensing electrode for sensing the inflow of blood, is connected with reference voltage source 65. Then, a certain voltage is applied between detection electrode 41 and detection electrode 42. In the state where blood flows in, a current flows between detection electrode 41 and detection electrode 42. This current is converted into a voltage by current/voltage converter 61 and this voltage value is converted into a digital value in A/D converter 62. The converted digital value is outputted to calculating section 63. Calculating section 63 detects based on the digital value that sufficient blood has flowed in. When the inflow of blood is detected, the operation of vacuuming means 56 is stopped.

When the inflow of blood is detected, according to a command from controlling section 66, switching circuit 60 is switched to connect detection electrode 41, which serves as an active electrode for measuring the amount of blood components, with current/voltage converter 61. Further, detection electrode 43 which serves as a counter electrode for measuring the amount of blood components, is connected with reference voltage source 65.

In the case where the blood component to measure is glucose, the glucose in blood and its oxidation-reduction enzyme may be reacted for a certain period. Preferably, during the reaction, current/voltage converter 61 and reference voltage source 65 suspend. After a certain reaction period passes, a certain voltage is applied between detection electrode 41 and detection electrode 43 according to the command from controlling section 61. The current that has flowed between detection electrode 41 and detection electrode 43 is converted into the voltage by current/voltage converter 61, and the voltage value is converted into a digital value by A/D converter 62 and is outputted toward calculating section 63. Calculating section 63 measures the amount of blood components (for example, glucose) based on this digital value.

After the amount of blood components is measured, an Hct (hematocrit) value is measured. First, switch circuit 60 is switched according to a command from controlling section 66, and then detection electrode 45, which serves as the active electrode for measuring the Hct value, is connected with current/voltage converter 61. Further, detection electrode 41, which serves as the counter electrode for measuring the Hct value, is connected with reference voltage source 63.

Next, according to a command from controlling section 66, a certain voltage is applied between detection electrode 45 and detection electrode 41 by current/voltage converter 61 and reference voltage source 65. The current flowing between detection electrode 45 and detection electrode 41 is converted into the voltage by current/voltage converter 61 and the voltage value is converted into a digital value by A/D converter 62. The digital value is outputted to calculating section 63 and calculating section 63 calculates an Hct value based on this digital value.

Based on a calibration curve or calibration curve table created in advance, the measured amount of glucose components is corrected by the calculated Hct value. The correction result is displayed in display section 64. Further, the correction result may be transmitted from communication section 67 to the injection apparatus that injects insulin. Although a radio wave may be used for this transmission, transmission is preferably performed by optical communication that does not interfere with medical equipment. Communication section 67 may transmit data related to the measurement test (i.e. the temperature when the test is carried out, the measurement test time, corrected data and data of the sensor type) in addition to the test result (or the corrected measurement result).

If the dose of insulin to administer is automatically set based on the correction result transmitted from communication section 67, patients need not to set the dose of insulin to administer. By this means, burden of setting is eliminated and it is possible to prevent setting errors. In this way, communication section 67 communicates data and the like with other equipment.

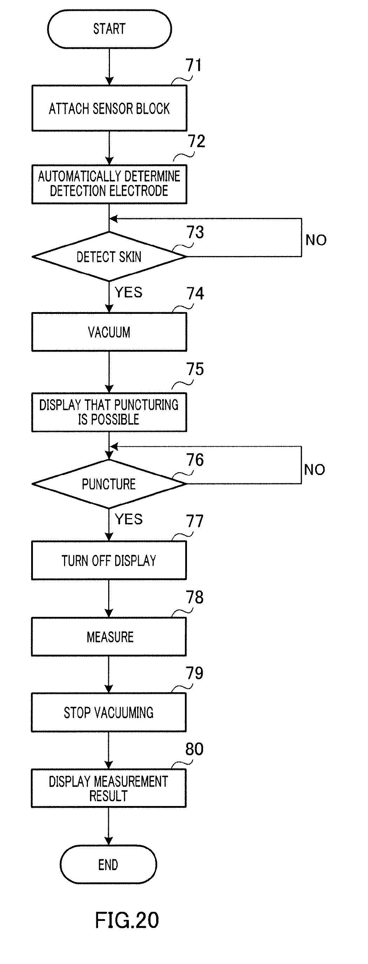

A measurement test flowchart by blood test apparatus 27 will be explained using FIG.20. In step 71, sensor block 21 is attached to insertion part 51b of cylindrical body 51a.

In step 72, by pressing the power supply switch of blood test apparatus 27 or by attaching sensor block 21, power is automatically supplied from battery 55 to electrical circuit section 52. When power is supplied to electrical circuit section 52, gears 54 are driven so that connectors 53a to 53f abut on connection electrodes 41a to 45a and reference electrode 43c, respectively. Next, reference electrode 43c of sensor 22 is specified. Based on specified reference electrode 43c, which one of detection electrodes 41 to 45 and reference electrode 43c each connector contacts is specified.

In step 73, stand-by continues until the blood sensor abuts on skin 9 to be punctured. When skin detecting sensors 23j of sensor block 21 detect contact of skin 9, vacuuming means 56 is operated in step 74. Vacuuming means 56 vacuums the vicinity of sensor 22. When a button for operating the vacuuming means is provided in controlling section 66 instead of arranging skin detecting sensors 23j, vacuuming means 56 is operated by pressing the button manually. When the value of the current that flows in the pump composing vacuuming means 56 changes or the predetermined time of vacuuming (the time is measured by timer 68) passes, it is decided that skin inside storing part 34 has been sufficiently sucked and lifted up.

In step 75, display section 64 displays that puncturing is possible. In step 76, according to this display, patients release an engagement with locking part 26b to puncture skin 9 with puncturing needle 25b. Blood flows out as a result of puncturing skin. Blood that has flowed out is taken in by detecting section 37 of sensor 22.

Display on display section 64 that puncturing is possible is turned off in step 77. That is, when blood reaches detection electrode 42, the display is turned off before blood components are measured (step 78). The vacuuming may be stopped at the same time.

In step 78, the amount of blood components taken in by detecting section 37 is measured. In step 79, the vacuuming by vacuuming means 56 is stopped. In step 80, the blood sugar level measured is displayed in display section 64. The order of step 79 and step 80 is not limited in particular, and either step may be carried out in advance or both steps may be carried out at the same time.

Although the blood test apparatus according to the present invention can measure glucose, the blood test apparatus is applicable to measure blood components other than glucose (for example, the lactate acid level or cholesterol).

### <Needle puncturing type blood test apparatus 2>

Another example of the needle puncturing type blood test apparatus will be explained below. This example of the blood test apparatus is characterized by having an attaching means that detachably attaches the sensor unit and needle unit individually to the insertion part for inserting the sensor block. The member for detachably attaching the sensor unit is referred to as the "first attaching part" and the member for detachably attaching the needle unit is referred to as the "second attaching part."

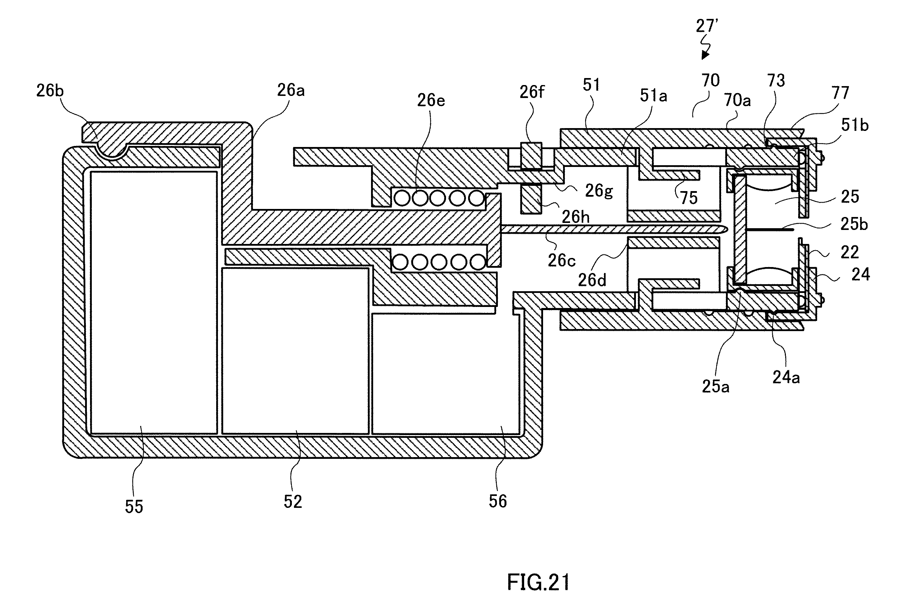

In blood test apparatus 27' shown in FIG.21, cylindrical body 51a of a cylindrical shape is formed at one end of housing 51 made of resin. Insertion part 51b is formed at the front end of cylindrical body 51a.

Needle unit 25 is inserted inside cylindrical body 51a from the insertion part 51b side. Needle unit 25 is locked by cylindrical body 51a through locking parts 251 and is attached in blood test apparatus 27'. On the other hand, sensor unit 24 fits onto the outer surface of cylindrical body 51a from the insertion part 51b side. Sensor unit 24 is locked by cylindrical body 51a through engaging parts 241 and is attached in blood test apparatus 27'.

Body part 70a composing attaching means 70 is provided slidably on the outer surface of cylindrical body 51a. First attaching part 73 composing body part 70a can detachably attach sensor unit 24, and attached sensor unit 24 can be pushed out and removed. Second attaching part 75 composing body part 70a can detachably attach needle unit 25, and attached needle unit 25 can be pushed out and removed.

Puncturing means 26 (including 26a to 26h) provided in housing 51 will be explained. Handle 26a is provided slidably in cylindrical body 51a. One end of handle 26a is guided outside housing 51 and is locked by locking part 26b. The other end of handle 26a is coupled to stick 26c facing needle unit 25. Stick 26c slides in guides 26d formed in practically the center of cylindrical body 51a. Handle 26a is urged toward needle unit 25 by springs 26e.

Adjusting part 26f for adjusting the puncturing depth has screw stick 26g and adjusting knob 26h, which is a nut meshing with screw stick 26g. By moving adjusting knob 26h by screwing in the left and right directions in FIG.21, to what extent handle 26a returns (i.e. the amount of movement toward insertion part 51b) is adjusted and the puncturing depth is adjusted.

Electrical circuit section 52 connected with blood sensor 22 in sensor unit 24 measures blood components (for example, blood sugar level) of blood 10 (see FIG.19) taken in by blood sensor 22. Vacuuming means 56 that vacuums the interior of cylindrical body 51a makes blood sampling easier by sucking and lifting up skin upon puncturing. Battery 55 is the electrical source for electrical circuit section 52.

The operation of blood test apparatus 27' shown in FIG.21 will be explained. Needle unit 25 is inserted inside cylindrical body 51a and is attached by engaging locking parts 251 with cylindrical body 51a. Next, sensor unit 24 fits onto the outer surface of cylindrical body 51a, and is attached by engaging locking parts 241 with cylindrical body 51a.

The connection electrodes provided in blood sensor 22 of attached sensor unit 24 contact the connectors provided in insertion part 51b in order to electrically connect blood sensor 22 and electrical circuit section 52.

Blood test apparatus 27' is made to abut on skin 9 (see FIG.3) to sample blood. Then, when the lock by locking part 26b is released, the restoring force of springs 26e makes stick 26c hit puncturing needle 25b of needle unit 25. Puncturing needle 25b passes the storing part of blood sensor 22 to puncture skin. Then, blood flows out from the punctured skin. Blood flowed out is taken in blood sensor 22, and generates a chemical reaction in blood sensor 22. Information about blood subjected to the chemical reaction is transmitted to electrical circuit section 52 through the connectors and the blood components are measured in electrical circuit section 52.

After the blood components are measured, sensor unit 24 and needle unit 25 are individually removed from attaching means 70 having first attaching part 73 and second attaching parts 75. When body part 70a moves toward insertion part 51b, first attaching part 73 presses against sensor unit 24, thereby releasing the lock by locking parts 241. By this means, sensor unit 24 is removed from cylindrical body 51a. Next, when body part 70a further moves toward insertion part 51b, second attaching parts 75 press against needle unit 25, thereby releasing the lock by locking parts 251. By this means, needle unit 25 is removed from cylindrical body 51a.

In this way, it is possible to remove sensor unit 24 with blood sensor 22 and needle unit 25 with puncturing needle 25b individually. Consequently, sensor unit 24 and needle unit 25 that have been used need not to be separated again and therefore puncturing needle 25b and sensor 22 that have been used can be separately discarded easily.

Further, sensor unit 24 and needle unit 25 can be individually removed and, even when one of sensor 22 and puncturing needle 25b is defective, only the defective one needs to be replaced, so that it is possible to prevent components without defect from being wasted.

Furthermore, after one blood component (for example, glucose) is measured, other blood components (for example, cholesterol and lactate acid) may be measured by replacing only sensor unit 24.

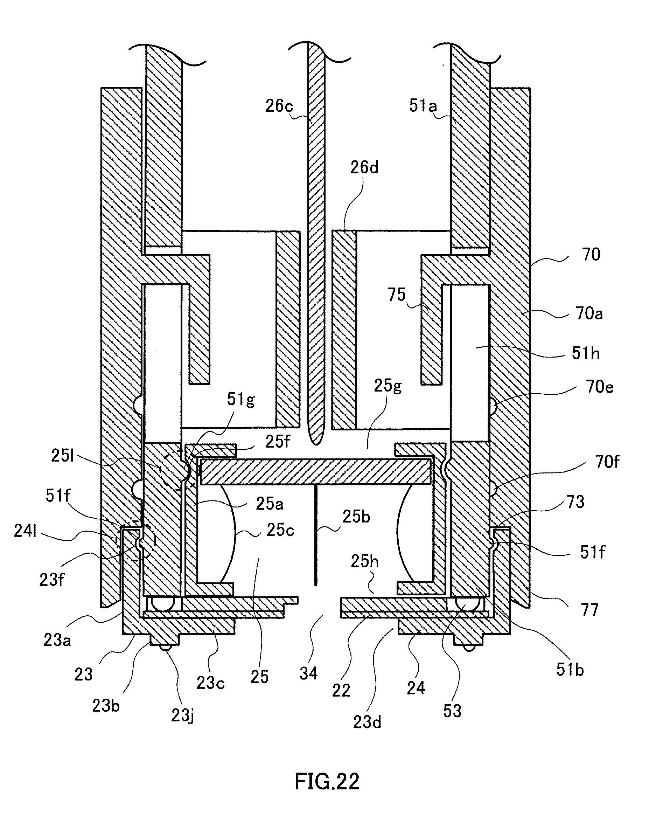

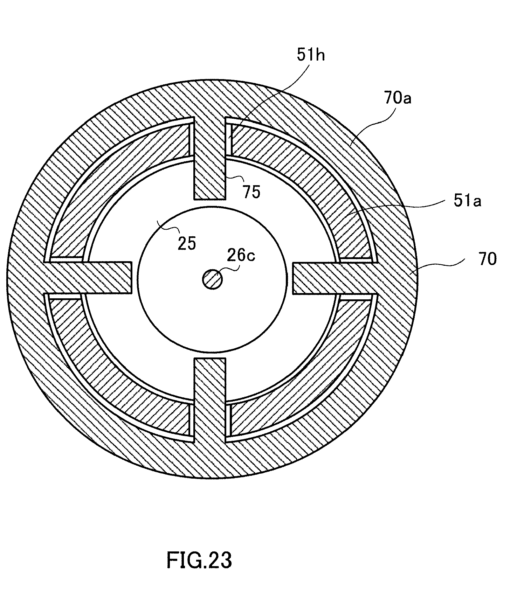

FIG.22 is a cross-sectional view near insertion part 51b of blood test apparatus 27' to which needle unit 25 and sensor unit 24 are attached. FIG. 23 is a plan view near insertion part 51b seen from above in FIG.22.

Sensor unit 24 is composed of: cylindrical holder 23 which is open on both ends; and blood sensor 22 which is attached to holder 23. Sensor unit 24 is made of resin material. Holder 23 is integrally formed with: cylindrical upper part 23a; cylindrical lower part 23b of a smaller diameter than upper part 23a; and circuit disc-shaped receiving part 23c which partitions between lower part 23b and upper part 23a and on which sensor 22 is arranged.

Positioning concave parts 23f are formed in the inner surface of upper part 23a. On the other hand, positioning convex parts 51f are formed in positions in the outer surface of insertion part 51b of cylindrical body 51a corresponding to positioning concave parts 23f of attached sensor unit 24. Concave parts 23f and convex parts 51f constitute locking parts 241. Hole 23d is formed in the center of receiving part 23c. Vacuuming means 56 vacuums the space defined by hole 23d and lower part 23b.

Skin detecting sensors 23j are arranged on the bottom surface of lower part 23b. The skin detecting sensors as a pair of conductive electrodes can be realized at low cost, but they may be optical sensors or temperature sensors. Skin detecting sensors 23j can be a pair of conductive electrodes provided in different positions in the bottom surface of lower part 23b.

Skin detecting sensors 23j are connected through copper interconnections with the electrodes formed in positioning convex parts 23f. When the resistance value changes between a pair of conductive electrodes upon contact with skin, the contact with skin is detected. Detection signals are communicated to electrical circuit section 52 through the electrodes in positioning concave parts 23f.

Needle unit 25 is composed of: cylindrical holder 25a made of resin; metal puncturing needle 25b of a thumbtack shape that is provided slidably in holder 25a; and elastic bodies that urge puncturing needle 25b upward. The elastic bodies are leaf springs 25c, coil springs or springs. Puncturing needle 25b is urged upward by leaf springs 25c, so that puncturing needle 25b is not exposed to the outside, which is safe.

Positioning concave parts 25f are formed in upper portions of holder 25a. Concave parts 25f and convex parts 51g of cylindrical body 51a constitute locking parts 251.

Circular hole 25g is provided above holder 25a of needle unit 25 and circular hole 25h is provided below holder 25a. Stick 26c passes hole 25g and hits puncturing needle 25b downward. By this means, puncturing needle 25b passes hole 25h and storing part 34 of blood sensor 22 to puncture skin.

Positioning convex parts 51g are formed in positions in the inner surface of cylindrical body 51a corresponding to positioning concave parts 25f of needle unit 25. Positioning convex parts 51g are elastic so as to engage with positioning concave parts 25f. Positioning concave parts 25f and positioning convex parts 51f constitute locking parts 251.

When needle unit 25 is inserted inside cylindrical body 51a, positioning concave parts 25f and positioning convex parts 51g are engaged, and thereby needle unit 25 is positioned in cylindrical body 51a. Next, when sensor unit 24 fits onto the outer surface of cylindrical body 51a, positioning concave parts 23f and positioning convex parts 51f are engaged, and thereby sensor unit 24 is positioned in cylindrical body 51a.

Connections 53 described later (including connectors 53a to 53f) are provided in insertion part 51b. When sensor unit 24 fits on cylindrical body 51a, connectors 53 contact with connection electrodes 41a to 45a (see FIG.13) formed in blood sensor 22. In this way, signals from connection electrodes 41a to 45a are supplied to electrical circuit section 52 through connectors 53. Signals from skin detecting sensors 23j are supplied to electrical circuit section 52 through the electrodes provided in positioning convex parts 51f.

Body part 70a composing attaching means 70 is a cylindrical member made of resin and can move slidably on the outer surface of cylindrical body 51a. First attaching part 73 for pushing out the upper end of sensor unit 24, is formed in the lower portion of body part 70a. Further, cover part 77 for covering the outer surface of holder 23 in sensor unit 24 is formed continuing to first attaching part 73. Preferably, cover part 77 has a tapered shape widening downward to make it easier to fit sensor unit 24 into the insertion part.

Second attaching parts 75 for pushing out needle unit 25 through cylindrical body 51b are provided in the inner surface of body part 70a. Second attaching parts 75 are members that branch out from body part 70a and have hook shapes. Four second attaching parts 75 are provided every 90 degrees (see FIG.23) in body part 70a. Further, concave parts 70e and concave parts 70f are formed in the inner surface of body part 70a. When attaching means 70 moves toward insertion part 51b and concave parts 70f come to the positions to engage with positioning convex parts 51f, first attaching part 73 removes sensor unit 24. When attaching means 70 further moves toward insertion part 51b and concave parts 70e come to the positions to engage with positioning convex parts 12f, second attaching parts 75 remove needle unit 25.

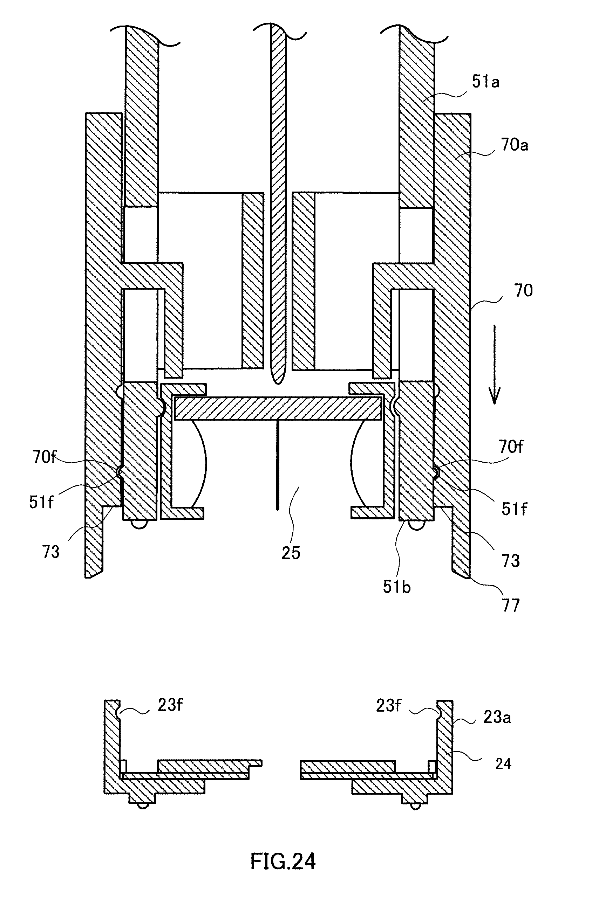

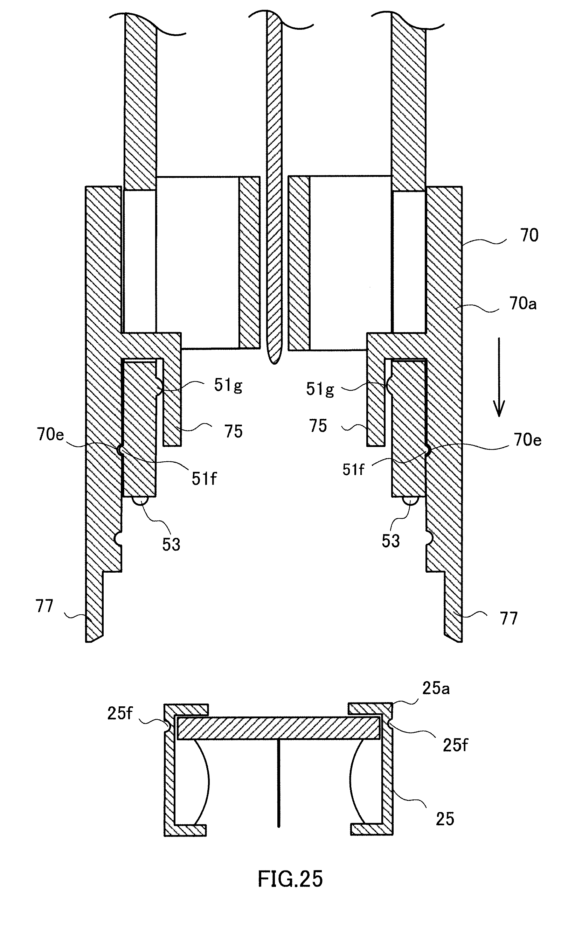

Removal of sensor unit 24 and removal of needle unit 25 will be explained using FIG.24 and FIG.25, respectively. Sensor unit 24 is removed follwed by needle unit 25.

FIG.24 shows a state where only sensor unit 24 is removed without removing needle unit 25. When body part 70a of attaching means 70 moves in the direciton of the arrow (i.e. toward insertion part 51b), the engagement between positioning concave parts 23f of blood sensor unit 24 and positioning convex parts 51f of cylindrical body 51a is released and thereby sensor unit 24 is removed. Further, concave parts 70f of body part 70a of attaching means 70 engage with positioning convex parts 51f, and body part 70a is also positioned accordingly.

When body part 70a further moves toward the direction of the arrow, second attaching parts 75 move in the direciton of the arrow and pushes out and removes needle unit 25. That is, as shown in FIG.25, the engagement between positioning concave parts 25f of needle unit 25 and positioning convex parts 51g of cylindrical body 51a is released. Further, concave parts 70e of body part 70a engage with positioning convex parts 51f, and thereby body part 70a is also positioned accordingly.

When sensor unit 24 is removed and when needle unit 25 is removed, cover part 77 provided at the front end of body part 70a also goes down. Cover part 77 that has gone down protects connectors 53 (including 53a to 53f) and prevents dust and dirt from adhering to connectors 53.

Sensor unit 24 and needle unit 25 are attached and removed in opposite steps. That is, to attach sensor unit 24 and needle unit 25, (1) needle unit 25 is inserted inside cylindrical body 51a and is positioned by engaging positioning concave parts 25f of needle unit 25 with positioning convex parts 51g of cylindrical body 51a. Next, (2) sensor unit 24 is fitted onto the outer surface of cylindrical body 51a. The upper end of holder 23 in sensor unit 24 abuts on insertion part 51b of cylindrical body 51a and pushes body part 70a of attaching means 70 upward. Sensor unit 24 is positioned by engaging positioning concave parts 23f of sensor unit 24 with positioning convex parts 51f provided in the outer surface of cylindrical body 51a.

Guides 23e (see FIG.7) are formed in the inner surface of upper part 23a of holder 23 in sensor unit 24. Guide grooves 51e (see FIG.10) are formed in the outer surface of cylindrical body 51a onto which sensor unit 24 fits. Even when cylindrical sensor unit 24 is inserted arbitrarily, guides 23e are guided along guide grooves 51e, so that it is possible to make connection electrodes 41a to 45a contact connectors 53a to 53f in a reliable manner.

Connectors 53 (including 53a to 53f) provided in insertion part 51b in blood test apparatus 27' need not to be moved to contact with connection electrodes 41a to 45a and realize reliable electrical connection.

With needle puncturing type blood test apparatus 27' shown in FIG.21, sensor unit 24 and needle unit 25 can be attached individually and removed individually. Consequently, even when one of sensor unit 24 and needle unit 25 is defective, the other one can be used. Therefore, sensor unit 24 and needle unit 25 can be separated easily and discarded.

Further, attaching means 70 of needle puncturing type blood test apparatus 27' shown in FIG.21 can remove sensor unit 24 and then remove needle unit 25, so that, for example, when blood sensor 22 is defective, it is possible to replace only blood sensor 22 easily. Further, by replacing only blood sensor 22 after puncturing, with another blood sensor, it is possible to measure many kinds of blood components (for example, cholesterol and lactate acid) using one needle unit 25.

### <Laser puncturing type blood test apparatus>

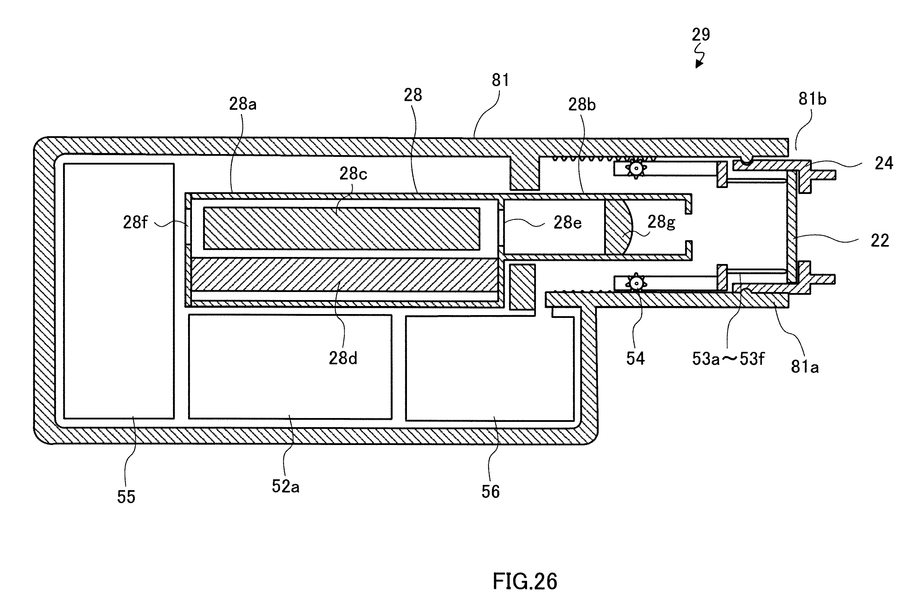

Next, the laser puncturing type blood test apparatus will be explained. FIG.26 is a cross-sectional view showing laser puncturing type blood test apparatus 29. Blood test apparatus 29 differs from blood test apparatus 27 shown in FIG.18 in that blood test apparatus 29 has laser emitting apparatus 28 as a puncturing means instead of a means for driving the needle unit, and in that only a sensor unit without a needle unit is attached to blood test apparatus 29 instead of a sensor block. The same members will be assigned the same reference numbers and explanation therefore will be simplified.

In FIG.26, one end of housing 81 made of resin is cylindrical body 81a of a cylindrical shape. Cylindrical body 81a composes insertion part 81b for sensor unit 24. Sensor unit 24 that removes needle unit 25 from sensor block 21 is inserted in insertion part 81b formed in the front end of cylindrical body 81a.

Laser emitting apparatus 28 is arranged facing blood sensor 22 attached to sensor unit 24. Guides 23g formed on the outer surface of holder 23 composing sensor unit 24 are inserted along the inner surfaces of guide grooves 81e formed in the inner surface of cylindrical body 81a (wherein guide grooves 81e correspond to guide grooves 51e in FIG.10). Then, positioning concave parts 23h formed in guides 23g fit in positioning convex parts 81f formed in deep inside guide grooves 81e and are positioned (where positioning convex parts 81f correspond to positioning convex parts 51f in FIG.5).

Laser emitting apparatus 28 has oscillating tube 28a and cylindrical body 28b of a cylindrical shape coupled to the front of oscillating tube 28a. Laser crystal 28c and flashing light source 28d are accommodated in oscillating tube 28a. Laser crystal 28c is, for example, Er:YAG (yttrium aluminum garnet). Partial transmission mirror 28e having about one percent of transmittance is attached to one end of oscillating tube 28a, and total reflection mirror 28f is attached to the other end.

Convex lens 28g is attached in cylindrical body 28b ahead of partial transmittance mirror 28e. Convex lens 28g is set to adjust focus of laser light on the surface of skins of patients. By setting the voltage of laser light to about 300 volt, it is possible to alleviate patients' pain.

The operation of laser emitting apparatus 28 will be explained. Light emitted from flashing light source 28d is radiated on laser crystal 28c. Light excited in laser crystal 28c is reflected between total reflection mirror 28f and partial transmission mirror 28e to oscillate, and is amplified. Part of amplified laser light passes partial transmission mirror 28e by stimulated emission. Laser light that has passed partial transmission mirror 28e passes lens 28g to be radiated and passes blood sensor 22 to radiate and puncture skin. Preferably, the depth that laser light from laser emitting apparatus 28 punctures into skin is between 0.05 mm and 1.5 mm and is, for example, 0.5 mm.

Blood test apparatus 29 with laser emitting apparatus 28 adopts a simple structure without movable components such as needle unit 25 with puncturing needle 25b and puncturing means 26 that need to be urged by springs, so that the number of components is small, and, consequently, it is easy to manage the components and failure of the apparatus is likely to decrease.

Further, blood test apparatus 29 can puncture skins of patients without contacting their skins and consequently is sanitary, and laser emitting apparatus 28 can employ a water-proof configuration, so that the apparatus can be washed entirely.

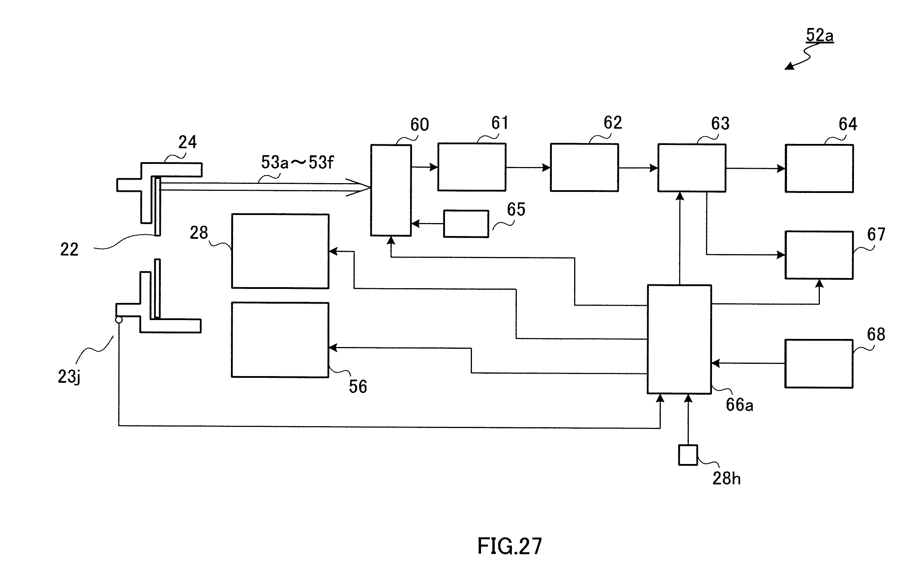

FIG.27 is a block diagram of electrical circuit section 52a. Electrical circuit section 52 is similar to electrical circuit section 52 shown in FIG.19 except that controlling section 66a controls laser emitting apparatus 28. That is, puncturing button 28h for commanding emission of laser light from laser emitting apparatus 28 is connected with controlling section 66a.

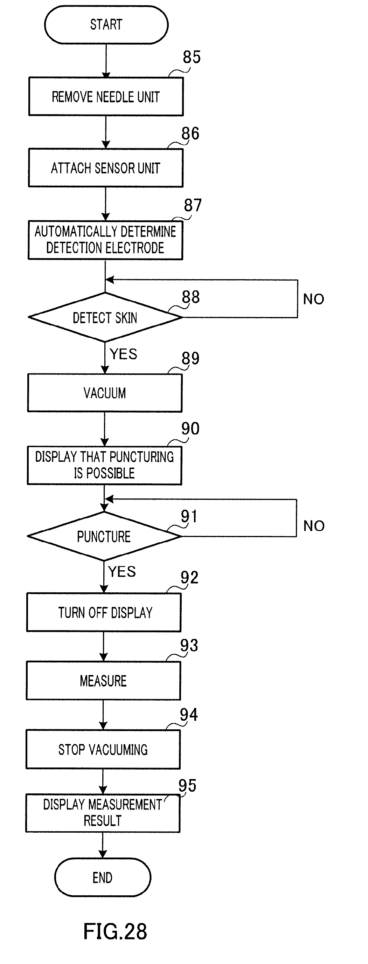

FIG.28 illustrates a test flowchart by blood test apparatus 29. First, in step 85, needle unit 25 is removed from sensor block 21 to obtain sensor unit 24. In step 86, sensor unit 24 is attached to insertion part 81b in cylindrical body 81a.

Step 87 to step 95 correspond to step 72 to step 80 in FIG.20. However, the puncturing means is laser emitting apparatus 28 and so puncturing button 28h is pressed instead of releasing engagement with locking parts 26b. That is, when puncturing button 28h is pressed, instead of driving puncturing needle 25b, laser light is emitted to puncture skin 9.

### <Blood test apparatus without puncturing means>

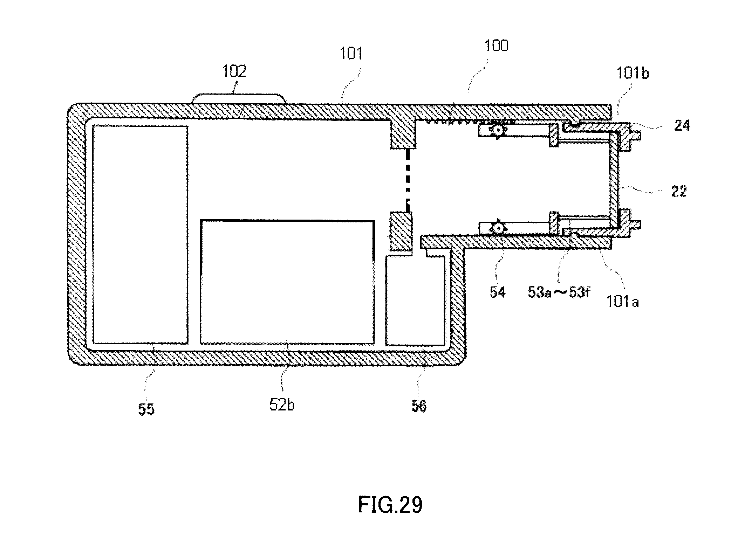

Next, the blood test apparatus without a puncturing means will be explained. FIG. 29 is a cross-sectional view of blood test apparatus 100 without a puncturing means. Blood test apparatus 100 differs from blood test apparatus 27 shown in FIG.18 in that blood test apparatus 100 does not mount a needle puncturing means and in that only a sensor unit without a needle unit is attached to blood test apparatus 100 instead of a sensor block. Further, blood test apparatus 100 differs from blood test apparatus 29 shown in FIG.26 in not mounting a laser puncturing means. The same members will be assigned the same reference numbers and explanation thereof will be simplified.

In FIG.29, one end of housing 101 made of resin is cylindrical body 101a of a cylindrical shape. Cylindrical body 101a forms insertion part 101b for sensor unit 24. Sensor unit 24 is inserted in insertion part 101b formed at the front end of cylindrical body 101a.

Guides 23g (see FIG.7B) formed in the outer surface of holder 23 composing sensor unit 24 are inserted along the inner surfaces of guide grooves (corresponding to guide grooves 51e in FIG.10) formed in cylindrical body 101a. Then, positioning concave parts 23h formed in guides 23g engaged with positioning convex parts (corresponding to positioning convex parts 51f in FIG.10) formed deep inside guide grooves formed in cylindrical body 101a, and are positioned.

A puncturing means is not mounted on blood test apparatus 100, and therefore electrical circuit section 52b does not have functions for controlling the puncturing means. Further, blood test apparatus 100 may or may not have vacuuming means 56. Connectors 53a to 53f contact connection electrodes 41a to 45a and 43c of blood sensor 22 to electrically connect blood sensor 22 and electrical circuit section 52b. Ejection button 102 works to eject sensor unit 24.

Blood test apparatus 100 does not have a puncturing means and therefore punctures skin using a separate puncturing tool. Blood sensor 22 attached to blood test apparatus 100 takes in blood that has flowed out from punctured skin, so that it is possible to measure blood components (i.e. it is possible to perform blood test).

As described above, by removing the needle unit from the sensor block to leave only the sensor unit, which sensor block can be applied to the needle puncturing type blood test apparatus, the sensor block can be used to the laser puncturing type blood test apparatus and blood test apparatus without a puncturing means. That is, the sensor block can be applied to any type of blood test apparatuses, so that blood sensors matching respective blood test apparatuses need not to be prepared, which is economical.

Further, a sensor unit having a blood sensor and a needle unit having a puncturing needle of the sensor block according to the present invention can be attached individually to a blood test apparatus and removed individually from the blood test apparatus, and, consequently, can be separately discarded easily.

The present invention claims priority based on Japanese Patent Application No.2006-344089, filed on December 21, 2006, and Japanese Patent Application No.2006-344090, filed on December 21, 2006. The disclosures including the specifications and drawings as filed, are incorporated herein by reference in their entirety.

### Industrial Applicability

The blood sensor block according to the present invention can be used in blood test apparatuses that perform puncturing by means of a puncturing needle and blood test apparatuses that perform puncturing by means of laser light, and can be applied to both blood test apparatuses. Further, by removing the needle unit from the blood sensor block according to the present invention to leave only the sensor block in the blood sensor block, the sensor block can be applied to blood test apparatuses without a puncturing means.

## Claims

1. A sensor unit comprising:
a holder; and
a blood sensor attached to the holder,
wherein a needle unit with a puncturing needle slidably provided can be attached detachably inside the holder in the sensor unit.

2. The sensor unit according to claim 1, wherein a shape of a cross section of the holder in the sensor unit is a circle, oval or polygon.

3. The sensor unit according to claim 1, wherein at least a portion of the holder in the sensor unit comprises a transparent member.

4. The sensor unit according to claim 1, wherein detecting means that detects contact with skin is provided in the holder in the sensor unit.

5. The sensor unit according to claim 1, wherein a surface of the holder in the sensor unit that contacts with skin is water-repellant.

6. The sensor unit according to claim 1, wherein the blood sensor in the sensor unit comprises:
a plurality of detection electrodes; and
a reference electrode for specifying the plurality of detection electrodes.

7. The sensor unit according to claim 1, wherein:
the blood sensor in the sensor unit comprises a blood storing part provided in practically the center of the blood sensor; and
a projecting part projecting from a blood supply channel is provided over the storing part.

8. The sensor unit according to claim 1, wherein the blood sensor and the holder in the sensor unit are formed integrally.

9. A blood sensor block comprising:
a sensor unit that comprises:
a holder; and
a blood sensor attached to the holder; and
a needle unit with a puncturing needle provided slidably,
wherein the needle unit is attached detachably to the holder in the sensor unit.

10. The blood sensor block according to claim 9, wherein:
a first insertion guide is provided in an inner surface of the holder in the sensor unit; and
the needle unit is attached in the holder in the sensor unit along the first insertion guide.

11. The blood sensor block according to claim 9, wherein the puncturing needle of the needle unit is urged in a direction to part from the blood sensor.

12. The blood sensor block according to claim 9, wherein:
a second insertion guide is provided in an outer surface of the holder in the sensor unit; and
the sensor unit is attached along the second insertion guide to a blood test apparatus that analyzes blood components based on a signal from the blood sensor.

13. A blood test apparatus comprising:
a housing that comprises at one end an insertion part to which the sensor unit according to claim 1 is attached;
an electrical circuit section that is provided in the housing and that is connected with a blood sensor in the sensor unit; and
puncturing means that is provided in the housing and that emits laser light to puncture skin.

14. The blood test apparatus according to claim 13, wherein the insertion part abuts on the blood sensor included in the sensor unit, and comprises a connector that electrically connects the electrical circuit section and the blood sensor.

15. The blood test apparatus according to claim 13, wherein:
a second insertion guide is provided in an outer surface of a holder in the sensor unit; and
a guide that meshes with the second insertion guide is provided in the insertion part.

16. The blood test apparatus according to claim 13, further comprising vacuuming means connected with the electrical circuit section.

17. The blood test apparatus according to claim 16, wherein:
detecting means that detecting contact with skin is provided in the holder in the sensor unit; and
the vacuuming means starts a vacuuming operation when the detecting means detects the contact with the skin.

18. The blood test apparatus according to claim 13, wherein the vacuuming means finishes a vacuuming operation when a detection electrode of the blood sensor detects an inflow of blood to the blood sensor.

19. The blood test apparatus according to claim 13, further comprising a communication function for transmitting a test result of blood test to external equipment.

20. A blood test apparatus comprising:
a housing that comprises at one end an insertion part to which the sensor unit according to claim 1 is attached; and
an electrical circuit section that is provided in the housing and that is connected with the blood sensor in the sensor unit,
wherein the blood test apparatus does not comprise puncturing means in the housing.

21. The blood test apparatus according to claim 20, further comprising a communication function for transmitting a test result of blood test to external equipment.

22. A blood test apparatus comprising:
a housing that comprises at one end an insertion part into which the blood sensor block according to claim 9 is attached;
an electrical circuit section that is provided in the housing and that is connected with the blood sensor in the sensor unit; and
puncturing means that is provided in the housing and that drives the puncturing needle of the needle unit to puncture skin.

23. The blood test apparatus according to claim 22, wherein the insertion part comprises a first attaching part and a second attaching part to which the sensor unit and the needle unit are detachably attached, respectively.

24. The blood test apparatus according to claim 23, wherein the second attaching part is arranged inside the first attaching part.

25. The blood test apparatus according to claim 22, wherein the sensor unit can be removed while the needle unit is kept attached.

26. The blood test apparatus according to claim 22, wherein the insertion part comprises a connector that abuts on the blood sensor included in the sensor unit, and that electrically connects the electrical circuit section and the blood sensor.

27. The blood test apparatus according to claim 22, wherein:
a second insertion guide is provided in an outer surface of the holder in the sensor unit; and
a guide that meshes with the second insertion guide is provided in the insertion section.

28. The blood test apparatus according to claim 22, further comprising vacuuming means connected with the electrical circuit section.

29. The blood test apparatus according to claim 28, wherein:
detecting means that detects contact with skin is provided in the holder in the sensor unit; and
the vacuuming means starts a vacuuming operation when the detecting means detects the contact with the skin.

30. The blood test apparatus according to claim 28, wherein the vacuuming means finishes a vacuuming operation when a detection electrode of the blood sensor detects an inflow of blood to the blood sensor.

31. The blood test apparatus according to claim 22, further comprising a communication function for transmitting a test result of blood test to external equipment.

32. A sensor unit comprising:
a holder; and
a blood sensor attached to the holder, wherein:
the sensor unit can be attached to the insertion part in the blood test apparatus according to one of claim 13, claim 20 and claim 22; and
a needle unit comprising a puncturing needle provided slidably can be attached detachably in the holder in the sensor unit.

33. A sensor unit comprising:
a holder; and
a blood sensor attached to the holder,
wherein the sensor unit can be attached to an insertion part of any one of:
a blood test apparatus comprising:
a housing that comprises at one end the insertion part to which the sensor unit is attached;
an electrical circuit section connected with the blood sensor in the sensor unit; and
puncturing means that is provided in the housing and that emits laser light to puncture skin; and
a blood test apparatus comprising:
a housing that comprises at one end the insertion part to which the sensor unit is attached; and
an electrical circuit section connected with the blood sensor in the sensor unit,
wherein the blood test apparatus does not comprise puncturing means in the housing; and
a blood test apparatus comprising:
a housing that comprises at one end the insertion part to which a blood sensor unit is attached;
a needle unit that comprises a puncturing needle provided slidably; and
puncturing means that drives the puncturing needle of the needle unit to puncture skin.

34. The sensor unit according to claim 33, wherein the blood sensor and the holder in the sensor unit are integrally formed.
